# EUROPÄISCHE PATENTSCHRIFT

(11) **EP 2 323 706 B1**
(45) Veröffentlichungstag und Bekanntmachung des Hinweises auf die Patenterteilung: **05.10.2016**
(21) Anmeldenummer: 09740840.5
(22) Anmeldetag: 28.08.2009
(51) Int. Cl.: A61L 27/30, A61L 27/56, A61L 27/58

(54) **DEGRADIERBARES IMPLANTAT UND VERFAHREN ZU SEINER HERSTELLUNG SOWIE DEREN VERWENDUNG**
DEGRADABLE IMPLANT AND METHOD FOR THE PRODUCTION THEREOF AND USE THEREOF
IMPLANT DÉGRADABLE ET PROCÉDÉ POUR SA PRODUCTION ET SON UTILISATION

(30) Priorität: 05.09.2008 DE 102008046197
(43) Veröffentlichungstag der Anmeldung: 25.05.2011
(73) Patentinhaber: Verein zur Förderung von Innovationen durch Forschung, Entwicklung und Technologietransfer e.V. (Verein INNOVENT e.V.), 07745 Jena (DE)
(72) Erfinder: HENNING, Angelika, 07751 Zöllnitz (DE); SCHNABELRAUCH, Matthias, 07749 Jena (DE); SCHRADER, Christian, 07743 Jena (DE); WEISSER, Jürgen, 99092 Erfurt (DE); SCHMIDT, Jürgen, 07745 Jena (DE); WYRWA, Ralf, 07751 Rothenstein (DE)
(74) Vertreter: Oehmke, Volker
(86) Internationale Anmeldenummer: PCT/DE2009/050048
(87) Internationale Veröffentlichungsnummer: WO 2010/025721

(56) Entgegenhaltungen:
- WO-A1-2006/004297
- US-A- 5 556 645
- US-A1- 2006 018 945

## Beschreibung

Die vorliegende Erfindung betrifft ein degradierbares Implantat, das wenigstens teilweise in den Knochen oder in andere Körperteile eingesetzt werden kann. Weiterhin betrifft die vorliegende Erfindung ein Verfahren zur Beschichtung eines degradierbaren Implantatgerüsts sowie deren Verwendung.

Die Entwicklung moderner Implantatwerkstoffe wurde in der jüngsten Vergangenheit durch die Anwendung von Materialien auf keramischer und biopolymerer Basis geprägt. Vor allem die letztere Gruppe wird im Allgemeinen als gut resorbierbar charakterisiert und erübrigt eine operative Entfernung derer nach dem abgeschlossenen Heilungsprozess.

Als weitere bioresorbierbare Werkstoffgruppe können Magnesium und Magnesiumlegierungen angesehen werden. Diese haben gegenüber abbaubaren Kunststoffen den entscheidenden Vorteil, dass sie wesentlich stabiler sind und über mechanische Eigenschaften verfügen, die denen des Knochenmaterials sehr ähnlich sind. So liegen E-Modul und Festigkeiten von Magnesium und Magnesiumlegierungen und Kortikalis sehr eng beieinander [Fertigung von Magnesium- Implantaten, F. Witte, C. Podolsky, T. Hassel, A. Lucas, wt Werkstatttechnik online Jg. 94 (2004) H. 11/12, S. 692 - 696; Magnesium Taschenbuch, C. Kammer, 1. Auflage, Düsseldorf, Aluminium- Verlag 2000]. Allerdings besteht bei der Verwendung von Magnesium und Magnesiumlegierungen als Implantatwerkstoff das Problem der raschen korrosiv bedingten Auflösung und des damit einhergehenden Freiwerdens von Korrosionsprodukten, wie z. B. molekularer Wasserstoff, der zur Bildung von Gastaschen im Gewebe führen kann (WO 02/100452 A1). Zur Lösung dieses Problems wurden in der Vergangenheit dem Magnesiumgrundwerkstoff Legierungskomponenten wie Aluminium (Al), Kalzium (Ca), Lanthan (La), Lanthanoide, Lithium (Li), Strontium (Sr), Yttrium (Y), Zink (Zn) und Zirkonium (Zr) zugesetzt. Diese verlangsamen den Korrosionsvorgang merklich und führen zu gleichmäßigen Abtragsraten. Frühere Untersuchungen konnten belegen, dass ein vollständiger Abbau des Implantatmaterials stattfindet, der Knochen in den ehemaligen Implantatbereich hinein wächst und sich das Magnesium (Mg) einschließlich seiner Legierungsbestandteile im Organismus ional auflöst, diffundiert und abtransportiert wird [Resorbierbares metallisches Osteosynthesematerial, in: Tätigkeitsbericht des Zentrum für Biomedizintechnik. F. Witte, V. Kaese, A. Meyer-Lindenberg, H. Windhagen, E. Switzer, A. Schiesler, I. Abeln, T. Fabian, T. Phantan, K. Philipp, M. Niemeyer, P. Wilk, H. Haferkamp; 1999-2001; S.10-21; Universität Hannover]. Der Hauptnachteil dieser gesteuerten Auflösung besteht darin, dass noch keine verlässlichen Daten zur toxischen Wirkung der verwendeten Legierungselemente vorliegen und sich aus diesem Grunde in naher Zukunft keine praxisreife medizinische Anwendung abzeichnet. Die Effekte des korrosionsschützenden Legierens von Mg-Li-, Mg-Al-Zn-, Mg-Li-Al-, Mg-Li-Al-Zn- sowie Mg-Li-Ca- Systemen, die verschiedenen wässrigen Korrosionseinflüssen ausgesetzt wurden, zeigen Möglichkeiten und Grenzen dieser Herangehensweise [Degradable, metallische Biomaterialien aus Magnesiumlegierungen für den Einsatz als Knochenimplantate, Prof. Dr. Carl Joachim Wirth, Medizinische Hochschule Hannover, Abteilung Orthopädie, MHH Forschungsbericht 2004, S. 309-316; Subtraction-XTM as a Sensitive Method for Detecting Rare Earth Elements in Magnesium Implants; F. Witte, J. Nellesen, H.A. Crostack, F. Beckmann; Hasylab Annual Report 2002, 2: 825-826; Characterization of a Magnesium Alloy as a Degradable Bone Implant Material by Synchrotron-based Microtomography; F. Witte, H.-A. Crostack , J. Nellesen, H. Windhagen, F. Beckmann; 18th European Conference on Biomaterials, October 1-4, 2003, Stuttgart].

Es ist bekannt, dass sich die Bioaktivität von Oberflächen metallischer Implantate durch eine Beschichtung mit Calciumphosphat wesentlich erhöhen lässt. Als bekannte und in der Technik eingeführte Verfahren, gelten dabei das Flamm- und Plasmaspritzen sowie die galvanischen Verfahren insbesondere die der anodischen Oxidation. In der WO 02/078759 A1 wird ein galvanostatisches Verfahren beschrieben, das zur Erzeugung anodisch-plasmachemischer Oberflächenmodifikationen führt. Ein entscheidender Nachteil dieser Methode besteht darin, dass die Calcium-phosphat (CaP) enthaltende Oberflächenschicht eine hohe Löslichkeit aufweist.

Calciumphosphat-Beschichtungen zeigen aufgrund ihrer chemischen Ähnlichkeit mit der mineralischen Knochenphase eine sehr gute Biokompatibilität. In Abhängigkeit von der Art der Calciumphosphat-Beschichtung und der damit verbundenen Löslichkeit können Calcium- und Phosphationen im neu gebildeten Knochen integriert werden. Dadurch wird ein stabilerer Verbund zwischen Implantat und umliegendem Knochengewebe ermöglicht. Eine optimierte offen-poröse Struktur der Calcium-phosphat-Beschichtung begünstigt zusätzlich das Einwachsen und die Verankerung von Knochenzellen. Diese als Osteokonduktivität bezeichnete Eigenschaft der Beschichtung fördert eine schnelle Stabilisierung des Implantats im umliegenden Gewebe.

Ein wesentlicher Nachteil von Calciumphosphat-Beschichtungen ist darin zu sehen, dass diese nicht osteogen wirken, also den Heilungsprozess des Knochengewebes nicht aktiv unterstützen. Daher wurden in den letzten Jahren vermehrt große Anstrengungen unternommen, Implantatoberflächen mit Substanzen zu beschichten, die eine osteogene oder osteoinduktive Wirkung entfalten können. Substanzen mit osteoinduktiver Wirkung sind beispielsweise Wachstumsfaktoren, wie die sogenannten Bone Morphogenetic Proteins (BMPs). Auch niedermolekulare Substanzen sind in der Lage, die Prozesse der Knochenbildung und der Knochenresorption zu beeinflussen. Als solche osteogene Substanzen sind aus der Literatur beispielsweise Bisphosphonate (z.B. Alendronat), Statine, wasserlösliche Strontiumsalze (z. B. Strontiumranelat) oder wasserlösliche Galliumsalze (z.B. Gallium(III)nitrat) bekannt. Gegenwärtig werden diese Substanzen in der Osteoporose-Behandlung teilweise bereits eingesetzt [Biskoping, D. M., Expert Opin. Invest. Drugs 12 (2003), 611-621; Bockman, R. S. et al., J. Bone Miner. Res. 4 (1989), 167]. Solche niedermolekularen Verbindungen sind zur Erzielung osteogener Eigenschaften von Implantatoberflächen sehr interessant, da sie im Vergleich zu Proteinen wie BMP in der Regel weniger empfindlich gegenüber etablierten Sterilisationsverfahren für Implantate sind. Unter den niedermolekularen Substanzen mit potentiell osteogener Wirkung sind Galliumsalze von besonderem Interesse. Bekannt ist, dass Galliumionen knochenbildende Zellen (Osteoblasten) aktivieren und eine anabole Aktivität (knochenwachstumsfördernd) besitzen. Auch inhibiert Gallium die Sekretion von IL-6 sowie anderen Osteoklasten aktivierenden Zytokinen. Die Knochenresorption durch knochenabbauende Zellen (Osteoklasten) kann durch Galliumionen reduziert werden, was höchstwahrscheinlich auf die Inhibierung vakuolärer ATPasen zurückzuführen ist [L. R. Bernstein: Pharmacological Reviews, 50, 4 (1998), 665-682]. Des Weiteren wurde gefunden, dass sich Gallium zur Behandlung tumorbedingter Hyperkalzämie eignet und die damit verbundene Calcium-resorption am Knochen reduziert wird [W. P. Raymond et al., J. Clin. Invest. (1984), 73, 1487-1490]. Wasserlösliche Galliumsalze wie Gallium(III)nitrat und Gallium(III)acetat sind daher als Pharmaka zur systemischen Applikation bei knochentumorbedingter Hyperkalzämie, Morbus Paget und Osteoporose [R. S. Bockman et al., Semin. Arthritis Rheum. 23 (1994), 268-269] sowie zur topischen Behandlung von Arthritis [G. Eby, Medical Hypotheses (2005) 65, 1136-1141] bereits klinisch getestet. Der Galliummetabolismus wurde aufgrund der Anwendung von ⁶⁷Ga in der Radionuclidmedizin bereits eingehend untersucht. Nachteilig wirkt sich bei einer oralen Gabe wasserlöslicher Galliumsalze die geringe Absorption im Intestinaltrakt aus. Es erfolgt nur eine geringe Aufnahme über die Duodenalschleimhaut und überwiegend ein Rücktransport in den Darm [P.O. Ganrot, Environmental Health Perspectives (1986), 65, 363-441]. Ein lokales Depot am Wirkungsort mit langsamer Freisetzung der Galliumionen wäre von großem Vorteil.

Es wurden in der Vergangenheit zahlreiche Versuche unternommen, die Osteointegration verbessernde hoch- oder niedermolekulare Substanzen auf unbeschichteten sowie auf Calciumphosphat- oder andersartig beschichteten Implantatoberflächen zu immobilisieren. Da eine rein adsorptive Immobilisierung auf Implantatoberflächen, beispielsweise durch Tauchen oder Besprühen derselben mit geeigneten Lösungen, der die Osteointegration verbessernden Substanzen den Nachteil einer schnellen Auswaschung der Substanzen aus der Oberfläche mit sich bringt, wurden unterschiedliche Verfahren einer dauerhaften Immobilisierung vorgeschlagen. Beispiele sind die kovalente Fixierung von Bisphosphonaten auf Calciumphosphat-Oberflächen [Peter B. et al., Local delivery of bisphosphonate from coated orthopedic implants increases implants mechanical stability in osteoporotic rats., J Biomed Mater Res A. 2006 Jan;76(1):133-143], die Beschichtung von metallischen Implantaten mit Suspensionen aus resorbierbaren Polymeren und BMP [Schmidmaier et al., Collective review: bioactive implants coated with poly(D,L-lactide) and growth factors IGF-I, TGF-betal, or BMP-2 for stimulation of fracture healing, J Long Term Eff Med Implants. 2006; 1 6(1):61-691. Wesentliche Nachteile all dieser Verfahren sind jedoch darin zu sehen, dass die Aufbringung der die Osteointegration verbessernden Komponente mit einem hohen technischen Aufwand verbunden ist und zusätzliche Hilfsstoffe wie Polymere, organische Lösungsmittel oder Kupplungsagenzien benötigt werden, die das Einwachsverhalten der Implantate negativ beeinflussen können. Zudem besitzen diese Schichten nur eine geringe mechanische Stabilität. Ferner bestehen außerdem Probleme bei der thermischen Behandlung oder γ-Bestrahlung zur Sterilisation, was zur Zerstörung der Beschichtung, Inaktivierung von Proteinstrukturen oder zu toxischen Abbauprodukten führen kann. Um diese genannten Probleme zu umgehen, wird in der US 5,556,645 A die Möglichkeit der Imprägnierung mit galliumhaltigen Verbindungen beschrieben. Jedoch können beim Tränken mit einer galliumhaltigen Lösung nur sehr dünne Schichten erhalten werden. Dickere Beschichtungen im µm-Bereich sind mit dem Imprägnieren nicht erreichbar. Zudem lassen sich Implantatoberflächen degradierbarer Metalle wie Magnesium nur schlecht imprägnieren. Da Gallium elektronegativer (edler) als Magnesium ist, würde sich Magnesium verstärkt auflösen. Mögliche Beschichtungen sind zudem nicht stabil und werden bei der mechanischen Scherbelastung einer Implantation, die im kPa bis MPa liegen kann, abgetragen, was zu einem undefinierten Gallium-Release führt und therapeutisch relevante Galliumionen-Dosen über längere Zeit nicht erreicht werden können. Durch eine Beschädigung der Beschichtung ist auch eine kontrollierte Degradation des Implantatmaterials nicht mehr gegeben.

Der Erfindung liegt die Aufgabe zugrunde, eine Möglichkeit anzugeben, die Degradierbarkeit von Implantaten zu steuern und die Gewebeverträglichkeit zu verbessern, wobei die Implantate, die wenigstens teilweise in den Knochen oder anderen Körperteilen eingesetzt werden, nach Ausheilung des versorgten Hart- bzw. Weichgewebes ihre Funktion während bzw. nach dem Heilungsprozess durch stattfindende Degradation verlieren.

Erfindungsgemäß wird die Aufgabe dadurch gelöst, dass eine auf einer Implantatstruktur festhaftende, poröse Beschichtung bereitgestellt wird, die als wirksamen Schichtbestandteil Galliumionen in Form eines variablen Anteils einer in wässrigen und/oder physiologischen Medien schwerlöslichen Galliumverbindung enthält. Die vorliegende Erfindung betrifft ein degradierbares Implantat bestehend aus einem Implantatgerüst und einer mindestens teilweise darauf befindlichen Beschichtung, dadurch gekennzeichnet, dass die Beschichtung auf dem Implantatgerüst Galliumionen in Form von in wässrigen und/oder physiologischen Medien schwerlöslichen Galliumverbindungen enthält, wobei der Galliumgehalt in der Beschichtung 1 bis 50 Atomprozente beträgt und die Galliumverbindungen in Form von überwiegend Galliumphosphat und/oder Gallium-Ca und/oder Mg-Phosphat vorliegen, wobei das Galliumphosphat in amorpher oder nanokristalliner Form vorliegt, eine Dicke von 0,1 bis 50 µm aufweist, und eine poröse Struktur mit einer Porendichte von 10⁴ bis 10⁷ Poren/mm² und einer durchschnittliche Porengröße zwischen 0,1 und 10 µm aufweist.

Vorteil der Erfindung ist es, dass bei der plasmachemischen Oxidation schon bei geringen Galliumionenkonzentrationen unter speziellen Bedingungen eine Anreicherung einer Galliumverbindung in Form von Galliumphosphat in der Beschichtung stattfindet und auch reine Galliumphosphatschichten generierbar sind.

Weiterhin von Vorteil ist es, dass dabei in den neuartigen galliumhaltigen Beschichtungen sich Poren bilden, die in einer Größenordnung von 0,1 bis 10 µm liegen und damit eine gesteuerte korrosive Auflösung der Implantatstruktur besonders effektiv ermöglicht wird.

Diese Beschichtungen zeichnen sich ferner dadurch aus, dass sie unabhängig vom Galliumgehalt von tierischen Zellen ohne Inhibition des Zellwachstums besiedelt werden. Es kann sich dabei auch um eine reine GaPO₄-Schicht handeln.

Die durch die Erfindung erreichten Vorteile sind im Wesentlichen darin zu sehen, dass Dank der erfindungsgemäßen porenhaltigen Beschichtung die Implantatstruktur wesentlich langsamer korrodiert. Dabei erfolgt durch die in der Beschichtung in Form von in Wasser schwerlöslichen Galliumverbindungen vorhandenen Galliumionen über lange Zeit eine Stimulierung knochenbildender Zellen (Osteoblasten), so dass ein verbesserter Heilungsprozess im angrenzenden Bereich gegeben ist. Da die Galliumionen in Form einer schwerlöslichen Galliumverbindung in die Beschichtung integriert sind, ist die Konzentration an gelösten, frei beweglichen Galliumionen in der Umgebung der Implantat-Grenzfläche gering, so dass zwar ausreichend Galliumionen zur Stimulierung des Knochenwachstums vorhanden sind, aber keine Gefahr einer Überdosierung und damit einer unerwünschten Nebenwirkung von Galliumionen auf das umgebende Gewebe besteht. Eine gewebeschädigende Konzentration oberhalb 50 µM wird praktisch nicht erreicht. Ein weiterer Vorteil der Beschichtung liegt in der entzündungshemmenden und antibakteriellen Wirkung von Galliumionen, die aus der Schicht langsam in das umliegende Gewebe freigesetzt werden.

Die poröse Beschichtung kann ebenfalls dazu dienen, in bekannter Weise Wirkstoffe bzw. Wirkstoffkombinationen - gegebenenfalls in einer Matrix eingebettet - aufzunehmen, wodurch zusätzlich z. B. wachstumsfördernde, antiseptische oder osteointegrationsfördemde Substanzen freigesetzt werden, sodass diese zusätzlichen bioaktiven Substanzen die entsprechenden Wirkungen unterstützend lokal entfalten. Der Galliumanteil in der Beschichtung beträgt 1 bis 50 Atomprozente, vorzugsweise 5 bis 30 Atomprozente.

Die Beschichtung enthält Galliumionen in Form von amorphem oder nanokristallinem Galliumphosphat, welches zusätzlich die Funktion von Kristallisationskeimen bei der Knochenneubildung besitzt.

In einer weiteren bevorzugten Ausführungsform der Erfindung enthält die Beschichtung amorphe und/oder kristalline Calciumphosphat-Phasen.

In einer weiteren Ausführungsform der Erfindung enthält die Beschichtung einen Anteil von 20 bis 95 Atomprozenten Metalloxid bestehend aus einem oder mehreren Metallen oder Mischungen unterschiedlicher Metalloxide.

Als besonders geeignetes Metalloxid hat sich Magnesiumoxid erwiesen.

Die Dicke der Beschichtung beträgt 0,5 bis 50 µm, vorzugsweise bis maximal 20 µm. Eine derartige, dünne Beschichtung verfügt über gute Hafteigenschaften auf dem Substrat und ist relativ stabil gegenüber mechanischer, insbesondere Scherbeanspruchung.

Ein wesentlicher Vorteil der Erfindung ist es, dass die Beschichtung porös ist, wobei die Porendichte zwischen 10⁴ und 10⁷ Poren/mm² und die durchschnittliche Größe der Poren zwischen 0,1 und 10 µm liegt.

Die erfindungsgemäße Beschichtung wird vorzugsweise auf Implantatgerüste aufgebracht, die aus Magnesium bzw. Magnesiumlegierungen bestehen und in der Medizin- und Dentaltechnik sowie in der Gefäßchirurgie eingesetzt werden. Die Implantatgerüste können dabei jede beliebige Form und Oberflächenmorphologie besitzen und die Beschichtung kann vollständig oder teilweise auf das Implantatgerüst aufgebracht werden.

Die erfindungsgemäße Beschichtung wird mittels anodischer Oxidationsverfahren, vorzugsweise dem Verfahren der plasmachemischen Oxidation auf die zu beschichtende Implantatstruktur aufgebracht. Der in diesem Verfahren eingesetzte wässrige Elektrolyt enthält dabei Gallium-, Phosphat- und gegebenenfalls weitere Ionen wie z.B. Calcium, welche Bestandteile der erfindungsgemäßen Beschichtung sein sollen, in Form geeigneter wasserlöslicher Salze. Der für die plasmachemische Oxidation verwendete Elektrolyt kann darüber hinaus weitere, den Prozess der Beschichtung verbessernde Zusätze wie Komplex- oder Chelatbildner oder den pH-Wert und/oder die Elektrolytleitfähigkeit einstellende Zusätze enthalten. Die Bildung der Beschichtung entsteht durch Reaktionen zwischen der metallischen Implantatstruktur und den Komponenten des Elektrolyten, unterstützt durch plasmachemische Reaktionen.

Ein wesentlicher, die Vorteile der Erfindung charakterisierender Aspekt besteht darin, dass der biologische Prozess der Wundheilung bzw. der Neubildung von Knochengewebe erheblich beschleunigt wird. Besonders die Anwesenheit von Galliumverbindungen aktiviert die Neubildung von Knochen und kann entzündliche Prozesse verringern.

Die vorliegende Erfindung beschreibt ein Verfahren zur Beschichtung von Implantaten aus Magnesium oder Magnesiumlegierungen, das es erlaubt ohne Verwendung von Seltenen Erden oder anderen speziellen Legierungselementen das Einsatzgebiet der z. Z. handelsüblichen Magnesiumguss- und -knetlegierungen als bioaktive und resorbierbare Implantate wesentlich zu erweitern. Es können aber auch Magnesiumlegierungen, die Seltene Erden, unterschiedliche Zinkgehalte und kein Aluminium enthalten, beschichtet werden.

Die Erfindung soll nachstehend anhand von Ausführungsbeispielen näher erläutert werden.

### Beispiel 1

In einer wässrigen Lösung, die 0,048 mol/l Etylendiamintetraessigsäure-Calcium-Dinatriumsalz-Hydrat, 0,104 mol/l Ammoniumdihydrogenphosphat, 0,088 mol/l Kaliumdihydrogenphosphat, 0,177 mol/l Phosphorsäure (85%), 2,465 mol/l Ammoniakwasser (25%), 1,641 mol/l Ethylendiamin (98%) und 0,011 mol/l Gallium(III)nitrat-Hydrat beinhaltet, wird die zu beschichtende Implantatstruktur aus der Magnesiumknetlegierung AZ31 als Anode geschaltet. Als Kathode dient ein elektrolytisch poliertes Edelstahlblech.

Mit einer Stromdichte von 2 A/dm² wird die Implantatstruktur bis zu einer Zellspannung von 300 V beschichtet. Es wird gepulster Gleichstrom mit einer Frequenz von 1 kHz verwendet. Die Implantatstruktur besitzt nach der Beschichtung eine Schicht mit einer mikroporigen Struktur und ist ca. 10 µm stark. Die Mikroporen weisen dabei einen Durchmesser von 0,2 bis 2 µm auf.

### Beispiel 2

In einer wässrigen Lösung, die 0,048 mol/l Etylendiamintetraessigsäure-Calcium-Dinatriumsalz-Hydrat, 0,104 mol/l Ammoniumdihydrogenphosphat, 0,088 mol/l Kaliumdihydrogenphosphat, 0,177 mol/l Phosphorsäure (85%), 2,465 mol/l Ammoniakwasser (25%), 1,641 mol/l Ethylendiamin (98%) und 0,011 mol/l Gallium(III)nitrat-Hydrat beinhaltet, wird die zu beschichtende Implantatstruktur aus der Magnesiumgusslegierung AZ91 als Anode geschaltet. Als Kathode dient ein elektrolytisch poliertes Edelstahlblech.

Mit einer Stromdichte von 2,5 A/dm² wird die Implantatstruktur bis zu einer Zellspannung von 320 V beschichtet. Es wird gepulster Gleichstrom mit einer Frequenz von 200 Hz verwendet. Die beschichtete Implantatstruktur besitzt nach der Beschichtung eine Schicht mit einer mikroporigen Struktur und ist ca. 12 µm stark. Die Mikroporen weisen dabei einen Durchmesser von 0,2 bis 1 µm auf.

### Beispiel 3

(Implantatstruktur aus einer Magnesiumlegierung, bestehend aus 89,9% Magnesium, 6% Zn, 2% SE, 1,5% Nd und 0,6% Zr)

In einer wässrigen Lösung, die 0,048 mol/l Etylendiamintetraessigsäure-Calcium-Dinatriumsalz-Hydrat, 0,104 mol/l Ammoniumdihydrogenphosphat, 0,088 mol/l Kaliumdihydrogenphosphat, 0,177 mol/l Phosphorsäure (85%), 2,465 mol/l Ammoniakwasser (25%), 1,641 mol/l Ethylendiamin (98%) und 0,011 mol/l Gallium(III)nitrat-Hydrat beinhaltet, wird die zu beschichtende Implantatstruktur aus einer Magnesiumlegierung, die Seltene Erden (SE) und Nd enthält, als Anode geschaltet. Als Kathode dient ein elektrolytisch poliertes Edelstahlblech.

Mit einer Stromdichte von 2 A/dm² wird die Implantatstruktur bis zu einer Zellspannung von 340 V beschichtet. Es wird gepulster Gleichstrom mit einer Frequenz von 250 Hz verwendet. Die beschichtete Implantatstruktur besitzt nach der Beschichtung eine Schicht mit einer mikroporigen Struktur und ist ca. 13 µm stark. Die Mikroporen weisen einen Durchmesser von 0,3 bis 2 µm auf.

### Beispiel 4

(Implantatstruktur aus einer Magnesiumlegierung, bestehend aus mindestens 96,6% Magnesium, 2% Y, 1% SE und jeweils kleiner gleich 0,2% Zn und Zr)

In einer wässrigen Lösung, die 0,048 mol/l Etylendiamintetraessigsäure-Calcium-Dinatriumsalz-Hydrat, 0,104 mol/l Ammoniumdihydrogenphosphat, 0,088 mol/l Kaliumdihydrogenphosphat, 0,177 mol/l Phosphorsäure (85%), 2,465 mol/l Ammoniakwasser (25%), 1,641 mol/l Ethylendiamin (98%) und 0,011 mol/l Gallium(III)nitrat-Hydrat beinhaltet, wird die zu beschichtende Implantatstruktur aus einer Magnesiumlegierung, die Yttrium und Seltene Erden enthält, als Anode geschaltet. Als Kathode dient ein elektrolytisch poliertes Edelstahlblech.

Mit einer Stromdichte von 4 A/dm² wird die Implantatstruktur bis zu einer Zellspannung von 270 V beschichtet. Es wird gepulster Gleichstrom mit einer Frequenz von 500 Hz verwendet. Die beschichtete Implantatstruktur besitzt nach der Beschichtung eine Schicht mit einer mikroporigen Struktur und ist ca. 5 µm stark. Diese Mikroporen weisen einen Durchmesser von 0,2 bis 2,5 µm auf.

Eine durchgeführte Röntgenfluoreszenzanalyse (RFA) der beschichteten Implantatstrukturen zeigten für die Gallium-Calcium-Phosphat (Ga-CaP) -Beschichtung deutliche Peaks für Gallium, Calcium und Phosphor. Für diese analytischen Untersuchungen wurden Magnesiumlegierungen verwendet, die den Ausführungsbeispielen 3 und 4 entsprechen und mit Seltenen Erden legiert waren.

Die Porosität der mittels anodischer Oxidation beschichteten Implantatstrukturen wurden mit Hilfe der Rasterelektronenmikroskopie (REM) untersucht. Dabei konnte festgestellt werden, dass die Oberfläche einer Ga-CaP-Beschichtung hauptsächlich Poren der Größen <1 µm enthält, wobei auch einzelne wenige Poren mit ca. 2 bis 5 µm je nach verwendeter Frequenz bei der Beschichtung auftraten.

Im Fluoresceindiacetat (FDA)/Ethidiumbromid (EtBr)-Test wurde die Cytotoxizität gegenüber tierischen Zellen bei direkter Besiedelung von erfingdungsgemäß hergestellten Implantaten nach 1 und 4 Tagen erfasst.

Für diese Tests wurden Zellen der Linie 3T3 (DSMZ ACC 173) eingesetzt. Die Kultur der Zellen erfolgte in DMEM/F12 (1:1) (Gibco) mit Zusatz von 10% fötalem Kälberserum und Antibiotika bei 37°C unter 5% Kohlendioxid. Zur Erfassung cytotoxischer Wirkungen wurden Implantate (der Abmessungen ca. 10 x 10 x 1 mm) mit 70% Ethanol desinfiziert, in Kavitäten einer 24-Well-Zellkulturplatte gelegt und mit phosphatgepufferter Kochsalzlösung (PBS) und Nährmedium gespült. Die Implantate wurden mit 3T3-Zellen in einer Zelldichte von ca. 25 000 Zellen/cm² beimpft und weiterkultiviert. Das Nährmedium wurde täglich erneuert (1 ml).

Nach 1 bzw. 4 Tagen wurden die Implantate entnommen und die Zellen mit einem Live/Dead-System, basierend auf Fluoresceindiacetat und Ethidiumbromid in PBS angefärbt. Die Auswertung erfolgte unter einem Fluoreszenzmikroskop. Im Vergleich dazu konnte nachgewiesen werden, dass unbeschichtete Implantatstrukturen stark cytotoxisch waren. Bereits nach einem Tag waren alle Zellen stark geschädigt und nach 4 Tagen nahezu alle Zellen tot. Durch die Beschichtung konnte die Cytotoxizität demgegenüber verringert werden. Bereits die Analyse nach dem ersten Tag verdeutlichte, dass die Schichten bessere Ergebnisse zeigten, als die unbeschichteten Implantatstrukturen. Nach dem viertem Tag ist dieses Ergebnis besonders augenfällig. Der Anteil toter Zellen liegt bei der Ga-CaP-Beschichtung bei ca. 15%, die Zellen wuchsen als adhärente Zellen mit typischer Morphologie an und zeigten eine deutliche Zunahme der Zelldichten im Zeitraum von 1 bis 4 Tagen.

## Patentansprüche

1. Degradierbares Implantat bestehend aus einem Implantatgerüst und einer mindestens teilweise darauf befindlichen Beschichtung, **dadurch gekennzeichnet, dass** die Beschichtung auf dem Implantatgerüst
- Galliumionen in Form von in wässrigen und/oder physiologischen Medien schwerlöslichen Galliumverbindungen enthält, wobei der Galliumgehalt in der Beschichtung 1 bis 50 Atomprozente beträgt und die Galliumverbindungen in Form von überwiegend Galliumphosphat und/oder Gallium-Ca und/oder Mg-Phosphat vorliegen, wobei das Galliumphosphat in amorpher oder nanokristalliner Form vorliegt,
- eine Dicke von 0,1 bis 50 µm aufweist, und
- eine poröse Struktur mit einer Porendichte von 10⁴ bis 10⁷ Poren/mm² und einer durchschnittliche Porengröße zwischen 0,1 und 10 µm aufweist.

2. Degradierbares Implantat nach einem der vorhergehenden Ansprüche, **dadurch gekennzeichnet, dass** die Beschichtung einen Anteil von 20 bis 95 Atomprozenten Magnesiumoxid enthält.

3. Degradierbares Implantat nach einem der vorhergehenden Ansprüche, **dadurch gekennzeichnet, dass** das Implantatgerüst aus Magnesium und/oder aus einer Magnesiumlegierung besteht.

4. Degradierbares Implantat nach einem der vorhergehenden Ansprüche, **dadurch gekennzeichnet, dass** die Oberfläche des Implantatgerüsts strukturiert und/oder porös ausgebildet ist.

5. Degradierbares Implantat nach einem der vorhergehenden Ansprüche, **dadurch gekennzeichnet, dass** in die Poren der Beschichtung ein Wirkstoff oder eine Wirkstoffkombination eingebracht ist und/oder in eine Matrix eingebettete Wirkstoffe eingebracht sind.

6. Verfahren zur Herstellung eines degradierbaren Implantats bestehend aus einem Implantatgerüst und einer mindestens teilweise darauf befindlichen Beschichtung nach einem der Ansprüche 1 bis 4, **dadurch gekennzeichnet, dass** das Implantat mindestens teilweise in einem wässrigen, Gallium- und Phosphationen enthaltenden Elektrolyten durch eine anodische Oxidationsreaktion beschichtet wird.

7. Verfahren nach Anspruch 6, **dadurch gekennzeichnet, dass** die anodische Oxidationsreaktion als plasmachemische Oxidation durchgeführt wird.

8. Verfahren nach Anspruch 7, bei dem
- ein wässriger Elektrolyt enthaltend:
- 0,012 bis 0,086 mol/l Etylendiamintetraessigsäure-Calcium-Dinatriumsalz-Hydrat,
- 0,05 bis 0,15 mol/l Ammoniumdihydrogenphosphat,
- 0,04 bis 0,1 mol/l Kaliumdihydrogenphosphat"
- 0,08 bis 0,25 mol/l Phosphorsäure (85%) und
- 0,5 bis 3,5 mol/l Ammoniakwasser (25%),
- 0,5 bis 2,4 mol/l Ethylendiamin (98%) und
- 0,01 bis 0,03 mol/l Gallium(III)nitrat-Hydrat
bereitgestellt wird,
- anschließend eine Edelstahlkathode eingeführt und elektrisch mit einer äußeren Stromversorgungsquelle kontaktiert wird,
- die zu beschichtende Implantatstruktur anodisch kontaktiert und mit der Stromversorgung verbunden wird,
- nach dem Einschalten der Stromversorgung, die einen gepulsten Gleichstrom mit einer Frequenz im Bereich von 10 bis 2000 Hz bei einem Tastverhältnis von 1:1 liefert, bei einer konstanten Stromdichte im Bereich von 2,0 bis 4,0 A/dm² die Badspannung (Startpunkt = 0 Volt) erhöht wird, wobei die Elektrolyttemperatur zwischen 10°C und 40 °C gehalten wird,
- bei erreichen der Badspannung von minimal 200 bis maximal 340 V wird die Spannung nicht weiter erhöht, infolge dessen es zu einem Absinken des Stromes kommt,
- bei erreichen der Hälfte des Ausgangswertes des Stromes wird der Beschichtungsprozess durch Ausschalten der Stromversorgung beendet und das fertig beschichtete Implantat wird aus dem Elektrolyt entfernt.

## Claims

1. Degradable implant composed of an implant structure and a coating that is located thereon at least partially, **characterized in that** the coating on the implant structure
- contains gallium ions in the form of gallium compounds that are sparingly soluble in aqueous and/or physiological media, wherein the gallium content in the coating amounts to 1 to 50 atom percent and the gallium compounds are present in the form of mainly gallium phosphate and/or gallium-Ca and/or -Mg phosphate, wherein the gallium phosphate is present in amorphous or nanocrystalline form,
- has a thickness of 0.1 to 50 µm, and
- has a porous structure with a pore density of 10⁴ to 10⁷ pores/mm² and an average pore size between 0.1 and 10 µm.

2. Degradable implant according to one of the preceding claims, **characterized in that** the coating contains a proportion of 20 to 95 atom percent of magnesium oxide.

3. Degradable implant according to one of the preceding claims, **characterized in that** the implant structure is composed of magnesium and/or of a magnesium alloy.

4. Degradable implant according to one of the preceding claims, **characterized in that** the surface of the implant structure is formed in a structured and/or porous manner.

5. Degradable implant according to one of the preceding claims, **characterized in that** an active substance or a combination of active substances and/or active substances embedded in a matrix are introduced into the pores of the coating.

6. Method for producing a degradable implant composed of an implant structure and a coating that is located thereon at least partially according to one of claims 1 to 4, **characterized in that** the implant is coated by an anodic oxidation reaction at least partially in an aqueous electrolyte containing gallium and phosphate ions.

7. Method according to claim 6, **characterized in that** the anodic oxidation reaction is realized as a plasma-chemical oxidation.

8. Method according to claim 7, in which
- an aqueous electrolyte containing:
- 0.012 to 0.086 mol/l ethylenediaminetetraacetic acid calcium disodium salt hydrate,
- 0.05 to 0.15 mol/l ammonium dihydrogen phosphate,
- 0.04 to 0.1 mol/l potassium dihydrogen phosphate,
- 0.08 to 0.25 mol/l phosphoric acid (85%) and
- 0.5 to 3.5 mol/l ammonia water (25%),
- 0.5 to 2.4 mol/l ethylene diamine (98%) and
- 0.01 to 0.03 mol/l gallium(III) nitrate hydrate
is provided,
- afterwards a stainless steel cathode is introduced and electrically contacted with an external power supply source,
- the implant structure to be coated is contacted anodically and linked with the power supply,
- after the power supply, which provides a pulsed direct current with a frequency in the range of 10 to 2000 Hz at a duty cycle of 1:1, has been switched on, the bath voltage (starting point = 0 volt) is increased at a constant current density in the range of 2.0 to 4.0 A/dm², wherein the electrolyte temperature is held between 10°C and 40°C,
- when the bath voltage of, at a minimum, 200 to, at a maximum, 340 V is reached, the voltage is not increased any further, resulting in a drop of the current,
- when half of the starting value of the current is reached, the coating process is terminated by switching off the power supply, and the finished coated implant is removed from the electrolyte.

## Revendications

1. Implant dégradable composé d'une structure d'implant et d'un revêtement se trouvant là-dessus au moins partiellement, **caractérisé en ce que** le revêtement sur la structure d'implant
- contient des ions gallium sous la forme de composés de gallium qui sont difficilement soluble dans des milieux aqueux et/ou physiologiques, le teneur en gallium dans le revêtement se montant à 1 à 50 pourcent d'atomes et les composés de gallium étant présents principalement sous la forme de phosphate de gallium et/ou phosphate de gallium-Ca et/ou -Mg, le phosphate de gallium étant présent sous forme amorphe ou nanocristalline,
- a une épaisseur de 0,1 à 50 µm, et
- a une structure poreuse avec une densité de pore de 10⁴ à 10⁷ pores/mm² et une taille de pore moyenne entre 0,1 et 10 µm.

2. Implant dégradable selon une des revendications précédentes, **caractérisé en ce que** le revêtement contient une proportion de 20 à 95 pourcent d'atomes d'oxyde de magnésium.

3. Implant dégradable selon une des revendications précédentes, **caractérisé en ce que** la structure d'implant est composée de magnésium et/ou d'un alliage de magnésium.

4. Implant dégradable selon une des revendications précédentes, **caractérisé en ce que** la surface de la structure d'implant est formée d'une manière structurée et/ou poreuse.

5. Implant dégradable selon une des revendications précédentes, **caractérisé en ce qu'**une substance active ou une combinaison de substances actives et/ou des substances actives encastrées dans une matrice sont introduites dans les pores du revêtement.

6. Procédé pour la production d'un implant dégradable composé d'une structure d'implant et d'un revêtement se trouvant là-dessus au moins partiellement selon une des revendications 1 à 4, **caractérisé en ce que** l'implant est revêtu par une réaction d'oxydation anodique au moins partiellement dans un électrolyte aqueux contenant des ions gallium et phosphate.

7. Procédé selon la revendication 6, **caractérisé en ce que** la réaction d'oxydation anodique est effectuée comme oxydation plasma-chimique.

8. Procédé selon la revendication 7, dans lequel
- un électrolyte aqueux contenant:
- 0,012 à 0,086 mol/l d'hydrate d'acide éthylène diamine tétraacétique-calcium-sel disodique,
- 0,05 à 0,15 mol/l de dihydrogénophosphate d'ammonium,
- 0,04 à 0,1 mol/l de dihydrogénophosphate de potassium,
- 0,08 à 0,25 mol/l d'acide phosphorique (85%) et
- 0,5 à 3,5 mol/l d'eau ammoniacale (25%),
- 0,5 à 2,4 mol/l d'éthylène diamine (98%) et
- 0,01 à 0,03 mol/l d'hydrate de nitrate de gallium(III)
est prévu,
- puis une cathode d'acier inoxydable est introduite et mise en contact électrique avec une source d'alimentation électrique externe,
- la structure d'implant à revêtir est contactée anodiquement et liée à l'alimentation électrique,
- après l'alimentation électrique, qui fournie un courant continu pulsé avec une fréquence dans la plage de 10 à 2000 Hz à un rapport cyclique de 1:1, a été mise en marche, la tension de bain (point de départ = 0 volt) est augmentée à une densité de courant constante dans la plage de 2,0 à 4,0 A/dm², la température d'électrolyte étant maintenue entre 10°C et 40°C,
- quand la tension de bain de 200, au minimum, à 340 V, au maximum, est atteinte, la tension n'est plus augmentée, et par conséquent, le courant est réduit,
- quand la moitié de la valeur de départ du courant est atteinte, le processus de revêtement est terminé en coupant l'alimentation électrique, et l'implant revêtu fini est retiré de l'électrolyte.
